# FASCICULE DE BREVET EUROPEEN

(11) **EP 2 217 698 B1**
(45) Date de publication et mention de la délivrance du brevet: **06.03.2013**
(21) Numéro de dépôt: 08843210.9
(22) Date de dépôt: 10.10.2008
(51) Int. Cl.: C12N 5/0783, C12N 5/0797

(54) **METHODE D'OPTIMISATION DES GREFFES DE CELLULES SANGUINES**
VERFAHREN FÜR OPTIMIERTE BLUTZELLENTRANSPLANTATION
METHOD FOR OPTIMIZING BLOOD CELL TRANSPLANTS

(30) Priorité: 12.10.2007 US 960761 P
(43) Date de publication de la demande: 18.08.2010
(73) Titulaire: Université Pierre et Marie Curie (Paris 6), 75005 Paris (FR); Assistance Publique Hôpitaux De Paris, 75004 Paris 4 (FR); Centre National de la Recherche Scientifique, 75016 Paris (FR)
(72) Inventeur: KLATZMANN, David, F-75013 Paris (FR); COHEN, José, F-75020 Paris (FR)
(74) Mandataire: Chajmowicz, Marion
(86) Numéro de dépôt international: PCT/FR2008/051844
(87) Numéro de publication internationale: WO 2009/053631

(56) Documents cités:
- EP-A- 0 972 518
- COHEN J L ET AL: "Would suicide gene therapy solve the ?T-cell dilemma? of allogeneic bone marrow transplantation?" IMMUNOLOGY TODAY, ELSEVIER PUBLICATIONS, CAMBRIDGE, GB, vol. 20, no. 4, 1 avril 1999 (1999-04-01), pages 172-176, XP004162793 ISSN: 0167-5699
- COHEN JOSE L ET AL: "Immunological defects after suicide gene therapy of experimental graft-versus-host disease" HUMAN GENE THERAPY, vol. 10, no. 16, 1 novembre 1999 (1999-11-01), pages 2701-2707, XP009116261 ISSN: 1043-0342
- CICERI FABIO ET AL: "Antitumor effects of HSV-TK-engineered donor lymphocytes after allogeneic stem-cell transplantation" BLOOD, vol. 109, no. 11, juin 2007 (2007-06), pages 4698-4707, XP002526048 ISSN: 0006-4971
- HOLLATZ ET AL: "T cells for suicide gene therapy: Activation, functionality and clinical relevance" JOURNAL OF IMMUNOLOGICAL METHODS, ELSEVIER SCIENCE PUBLISHERS B.V.,AMSTERDAM, NL, vol. 331, no. 1-2, 21 décembre 2007 (2007-12-21), pages 69-81, XP022481130 ISSN: 0022-1759
- MAURY S ET AL: "Effect of combined cytostatic cyclosporin A and cytolytic suicide gene therapy on the prevention of experimental graft-versus-host disease" GENE THERAPY, vol. 9, no. 3, février 2002 (2002-02), pages 201-207, XP002526506 ISSN: 0969-7128

## Description

La présente invention concerne le domaine des greffes de cellules souches hématopoïétiques. De façon plus précise, cette invention concerne le conditionnement de receveurs de cellules souches hématopoïétiques avec une composition contenant des lymphocytes T pour optimiser la greffe des cellules souches hématopoïétiques.

Les cellules souches hématopoïétiques sont des cellules à l'origine de toutes les lignées de cellules sanguines. Ces cellules sont capables de donner naissance en se différenciant, à n'importe quelle cellule du sang (globules rouges, globules blancs, plaquettes) et sont aussi capables d'auto- renouvellement.

La greffe de cellules souches hématopoïétiques est devenue ces dernières années, un des moyens thérapeutiques majeurs dans le traitement de certaines maladies du sang et de certains cancers. En effet, elle rend possible une intensification thérapeutique par chimiothérapie et/ou de radiothérapie à doses massives ayant pour résultat le traitement de la maladie, voire sa guérison avec une amélioration de la survie du patient.

Une greffe effectuée après ces traitements qui comportent une toxicité hématologique importante, permet la reconstruction de la moelle osseuse et le retour à une production normale de cellules sanguines.

La greffe de cellules souches hématopoïétiques intervient dans le traitement de plusieurs types de pathologies : malignes comme les leucémies aigues, les myélomes, les lymphomes ou certaines tumeurs solides (cancer du sein, neuroblastome) mais aussi non malignes comme les déficits constitutionnels ou acquis (déficit immunitaire, aplasies, erreurs métaboliques).

Il existe trois sources de cellules souches hématopoïétiques : la moelle osseuse, le sang périphérique et le sang placentaire. Deux types de greffe peuvent être pratiquées :
- l'autogreffe où le patient reçoit ses propres cellules souches prélevées dans la moelle osseuse, le sang de cordon ou dans le sang périphérique plusieurs semaines, mois ou années auparavant et conservées congelées.
- l'allogreffe qui nécessite un donneur familial ou non apparenté.

La greffe s'effectue selon plusieurs étapes. Quelques jours avant de subir la greffe, le patient est généralement soumis à un « conditionnement » myéloablatif ou non myéloablatif. Il s'agit souvent d'une chimiothérapie forte et éventuellement une irradiation totale pour détruire la moelle du receveur et permettre la prise de greffe. Le but est de détruire toutes les cellules présentes dans les cavités médullaires du receveur pour permettre aux cellules souches hématopoïétiques du donneur de s'y développer et se substituer ainsi à la moelle osseuse détruite du receveur.

La greffe de cellules hématopoïétiques s'effectue ensuite par transfusion intraveineuse. La greffe est suivie par une période d'aplasie pendant laquelle le patient n'a plus de défenses immunitaires.

La période de « conditionnement » précédent la greffe permet d'obtenir un effet antitumoral sur les cellules malignes et de créer une immunosupression suffisante pour empêcher le rejet du greffon. Un effet important dans la prise de greffe est lié à la présence ou à l'absence de lymphocytes T allogéniques dans le greffon. Ces lymphocytes T, via une activation allogénique favorisent la prise de greffe en détruisant des cellules immunocompétentes résiduelles du receveur, évitant ainsi leur action de rejet des cellules greffées. Toutefois, cet effet bénéfique est contrebalancé par les risques élevés de maladie du greffon contre l'hôte (GVH : Graft versus host). C'est la complication principale et limitante de la greffe de moelle allogénique. Bien que le mécanisme soit encore partiellement incompris, la GVH est liée à l'activation des lymphocytes T matures qui reconnaissent les antigènes de l'hôte comme des antigènes étrangers. L'activité cytotoxique qui en résulte est alors directe (action des lymphocytes cytotoxiques) ou indirecte par recrutement d'autres cellules effectrices et sécrétion de cytokines. La GVH aigue survient généralement dans les 2 à 5 semaines qui suivent la greffe et peut avoir une fréquence comprise entre 10 et 80% des cas selon la disparité génétique entre donneur et receveur. On parle de GVH chronique quand elle intervient plus de 100 jours après la greffe.

Une solution habituellement utilisée pour prévenir la GVH est d'administrer au patient la molécule immunosuppressive cyclosporine A (CsA) 3 à 6 mois suivants la greffe de cellules souches hématopoïétiques (Storb R et al, 1989). Mais le traitement n'est que partiellement efficace puisque 15 à 60% des patients vont développer une GVH (Socie et al, 1998). L'utilisation d'autres molécules immunosupressives telles que FK506 n'a pas induit la diminution de la fréquence de la GVH (Ratanatharathorn et al, 1998). Une autre stratégie pour prévenir la GVH est de faire une greffe de cellules souches hématopoïétiques déplétées de lymphocytes T (Aversa et al 1998). L'inconvénient majeur de cette stratégie est qu'elle ne permet pas la reconstitution satisfaisante des lymphocytes T ce qui engendre des complications dues à des infections. Une autre solution a été proposée dans le cadre d'une greffe de moelle osseuse allogénique, consistant à supplémenter le greffon de lymphocytes T exprimant le gène suicide de la thymidine kinase (TK) qui permet l'élimination par le gancyclovir des lymphocytes T qui l'expriment (Cohen et al 2001). Dans ce cas, le gancyclovir était administré 7 jours après la greffe de moelle osseuse comprenant des lymphocytes T transduits avec le gène de la thymidine kinase.

### RESUME DE L'INVENTION :

Les inventeurs proposent maintenant d'utiliser, lors du « conditionnement » du patient avant la greffe de cellules souches hématopoïetiques, des lymphocytes T exprimant une molécule permettant leur destruction spécifique, dans le but de favoriser la prise de greffe.

Est ici décrite l'utilisation de lymphocytes T allogéniques, pour la préparation d'une composition destinée à être injectée à un patient receveur avant une (en conditionnement d'une) greffe de cellules souches hématopoïetiques, lesdits lymphocytes T allogéniques exprimant une molécule permettant leur destruction spécifique.

Un objet de l'invention concerne une composition de lymphocytes T allogéniques, pour à une utilisation dans le conditionnement d'un patient receveur d'une greffe de cellules souches hématopoïetiques, par injection de la composition audit patient receveur avant la greffe, lesdits lymphocytes T allogéniques exprimant une molécule permettant leur destruction spécifique.

Est également décrite une méthode pour prévenir le développement de la maladie GVH chez un patient qui va subir une greffe de cellules souches hématopoïétiques, la méthode comprenant :
- le conditionnement du patient par un traitement lymphopéniant, myéloablatif ou non myéloablatif,
- associé avec une injection de lymphocytes T exprimant une molécule permettant leur destruction spécifique;
- puis, éventuellement, la destruction des lymphocytes T dans les trois jours.

Cette méthode permet de diminuer le risque de développement de la maladie GVH. Elle permet également d'optimiser le conditionnement du receveur et donc de favoriser la prise de greffe, et d'injecter des Lymphocytes T pouvant provenir d'un fond génétique différent de celui du donneur et du receveur.

La méthode permet par ailleurs d'utiliser une plus faible quantité de cellules souches hématopoïétiques, rendant possible l'utilisation de cellules souches d'un seul sang de cordon.

### DESCRIPTION DETAILLEE DE L INVENTION:

### Définitions

Les lymphocytes T expriment une « molécule permettant leur destruction spécifique ». Il peut s'agir d'une molécule codée par un transgène ou une molécule exprimée naturellement par les lymphocytes T. Le terme «destruction spécifique» signifie que seuls les lymphocytes T administrés au patient seront détruits, pour empêcher le développement d'une réaction de GVH.

La « molécule permettant leur destruction spécifique » peut être par exemple un antigène du système HLA, les molécules Thy-1, récepteur NGF ou une forme tronquée du récepteur, ou encore un antigène non-immunogène et non exprimé naturellement par les lymphocytes T. Les lymphocytes T portant l'une ou l'autre de ces molécules peuvent alors être détruits spécifiquement par un sérum anti-lymphocytaire, ou des anticorps dirigés spécifiquement contre lesdits antigènes.

La « molécule permettant la destruction spécifique » des lymphocytes T peut également être une molécule codée par un « gène suicide ».

Le terme « gène suicide » se réfère à un gène codant pour une molécule toxique pour la cellule qui l'exprime, de manière conditionnelle.

### Sources des lymphocytes T

Les lymphocytes T allogéniques peuvent provenir du donneur de CSH ou d'un donneur différent, qui n'est ni le donneur ni le receveur.

Le donneur de lymphocytes T est de préférence humain, et peut être un foetus, un nouveau-né, un enfant, un adulte.

Les préparations de lymphocytes T sont obtenues par exemple à partir de sang périphérique, du produit sanguin d'une lymphaphérèse, de ganglions lymphatiques périphériques, de la rate, du thymus, du sang de cordon, etc.

L'injection des lymphocytes T est réalisée pour préparer la greffe de cellules souches hématopoïétiques. L'injection des lymphocytes T est réalisée de 1 à 15 jours, et plus préférentiellement de 3 à 10 jours avant la greffe de cellules souches hématopoïétiques.

### Transduction de molécules permettant la destruction spécifique des lymphocytes T:

### Transduction de molécules permettant la destruction spécifique des lymphocytes T :

Dans un mode de réalisation particulier, les lymphocytes T sont modifiés de manière à exprimer un transgène codant pour une molécule permettant la destruction spécifique desdits lymphocytes T.

De manière préférentielle, le transgène est un gène «suicide». Par exemple il peut s'agir d'un gène qui code pour une molécule apte à phosphoryler un analogue de nucléoside en une molécule monophosphatée, elle-même convertible par des enzymes cellulaires en nucléotide triphosphaté incorporable dans des acides nucléiques en cours d'élongation sous l'effet des polymérases avec pour effet l'interruption d'élongation des chaînes. Ledit analogue de nucléoside peut être par exemple l'acyclovir ou le gancyclovir. Ladite molécule exprimée par le gène « suicide » peut être notamment une thymidine kinase, choisie par exemple parmi la thymidine kinase (TK) du virus de l'herpès simplex de type 1, la thymidine kinase du virus herpes équin, ou une thymidine kinase tronquée (voir notamment le brevet EP1046400). On utilise avantageusement la thymidine kinase (TK) du virus de l'herpès simplex de type 1.

La thymidine kinase du virus de l'herpès simplex 1 (HSV1-TK), est apte, lorsqu'elle est présente en une concentration suffisante dans les cellules en cause, à phosphoryler des analogues de nucléosides, tels que l'acyclovir (9-t(2-hydroxyethoxy)méthyl]guanine) ou le gancyclovir (9-[1,3-lOhydroxy-2- propoxyméthyl]guanine), en des molécules monophosphatées, elles-mêmes convertibles par des enzymes cellulaires en nucléotides triphosphatés incorporables dans des acides nucléiques en cours d'élongation sous l'effet des polymérases au sein desdites cellules, avec pour effet l'interruption d'élongation de chaînes et la mort cellulaire qui s'ensuit.

En cas de GVH, on administre alors au patient l'analogue de nucléoside (par exemple le gancyclovir).

De manière avantageuse, on administre l'analogue de nucléoside (par exemple, le gancyclovir) de manière préventive avant la greffe, par exemple dans les 3 jours après l'injection des lymphocytes T (de préférence de 3 à 10 heures après).

On peut avoir recours à toute technique adéquate pour transférer le transgène, notamment par infection in vitro des cellules correspondantes par une pseudo-particule virale de type Moloney amphotrope. Ces particules virales sont produites par une lignée cellulaire dite de "packaging" que l'on aura construite au préalable. Une lignée de packaging est capable de fabriquer tous les éléments structuraux constituant une particule virale, mais est incapable d'introduire dans des particules virales en voies de maturation les ARNs viraux produits par cette lignée cellulaire. C'est pourquoi, ces lignées dites de packaging fabriquent en permanence des particules virales vides. L'introduction d'une construction génétique appropriée, qui contient l'ADN recombinant tel qu'il a été défini plus haut, permet à ces lignées de packaging d'être introduites dans les particules virales vides réalisant ainsi des pseudo-particules virales. Ces pseudo-particules virales sont capables d'infecter différentes cellules cibles, cellules cibles qui varient selon la lignée de packaging qu'on a utilisée au départ. Par exemple, si cette lignée de packaging est dérivée d'un virus dit de Moloney amphotrope, les particules virales produites infectent parfaitement les cellules hématopoïétiques humaines.

Les techniques classiques pour la production de lignées de cellules transformées par un vecteur rétroviral (voir par exemple Danos et al, 1988 et Markowitz et al, 1988), peuvent être transposées à la production de lymphocytes. De même les techniques de transfert génétique mettant en oeuvre de tels systèmes (Kasid et al, 1990) peuvent être appliquées au transfert, chez l'homme, des cellules telles qu'elles ont été définies plus haut. Le transfert peut également être réalisé selon la méthode décrite dans Lemoine et al. (Efficient transduction and selection of human T-lymphocytes with bicistronic Thy1/HSV1-TK retroviral vector produced by a human packaging cell line, J Gene Med. 2004 Apr;6(4):374-86). Selon un autre mode particulier de réalisation de l'invention, les lymphocytes T peuvent être modifiés génétiquement en utilisant un vecteur rétroviral SFCMM-2 codant pour le gène « suicide » de fusion HSV-TK/Neo, selon le procédé de transduction décrit dans Ciceri et al, 2007.

### Patient

Le patient visé est un humain, quel que soit son âge et son sexe, qui va subir une greffe de cellules souches hématopoïétiques. Le patient soufre de toute maladie pouvant être traitée par une greffe de CSH. Il s'agit notamment de cancers, de maladies génétiques, de maladies qui affectent le système hématopoïétique et/ou le système immunitaire. On peut notamment citer : les tumeurs solides, les hémopathides malignes, dont la leucémie myéloïde chronique, la leucémie myéloïde aiguë, la leucémie lymphoblastique aiguë, les syndromes myéloprolifératifs, les syndromes myélodysplasiques, les LNH (lymphomes malins non hodgkiniens), le Hodgkin, l'aplasie médullaire sévère idiopathique, l'hémoglobinurie nocturne paroxystique, les hémoglobinopathies sévères, les déficits immunitaires congénitaux sévères (SCID, Kostmann, Wiskott-Aldrich) et l'anémie de Fanconi.

### Conditionnement du patient

Le conditionnement du patient, associé à l'injection des Lymphocytes T exprimant le gène « suicide » avant la greffe de cellules souches hématopoïétiques, peut être un traitement lymphopéniant myéloablatif ou non myéloablatif.

Une revue de ce type de traitement est présentée dans Petersen et al, 2007. Un exemple de traitement lymphopéniant myéloablatif peut être le suivant : la cyclophosphamide (120mg/kg) combinée avec du busulfan (16mg/kg) ou avec une irradiation totale du patient avec une dose de rayon absorbée de 12 Gy (« total body irradiation »).

Dans le cas du traitement lymphopéniant non-myéloablatif, le patient peut être totalement irradié avec dose de radiation absorbée de 2Gy ou recevoir une chimiothérapie à base de cyclophosphamide et/ou fludarabine. Par exemple Miller et al, 2007, décrivent un traitement lymphopéniant non myéloablatif, qui comprend une injection intraveineuse de cyclophosphamide 50mg/kg une fois à J-6 et J-5, et injection de fludarabine 25mg/m² de J-6 à J-2 (J0 étant le jour de la greffe de cellules souches hématopoïétiques).

### Les cellules souches hématopoïétiques

Les cellules souches hématopoïétiques utilisées peuvent être issues de n'importe quelle source, par exemple du sang périphérique, de la moelle osseuse ou du sang de cordon ombilical.

En effet, la méthode proposée permet de diminuer le nombre de CSH à injecter pour qu'il y ait prise de greffe.

Dans un mode de réalisation préféré, on utilise moins de 2 à 3x10⁷ cellules nuclées de sang de cordon ombiliical par kg de patient greffé. Lorsque les cellules souches proviennent de la moëlle ou du sang periphérique, on utilise moins de 2x10⁸ cellules nuclées par kg (de patient greffé) ou moins de 2-3x10⁶ cellules CD34+ par kg (de patient greffé).

### Protocole

Puisque les lymphocytes T expriment une molécule permettant leur destruction spécifique, la GVH peut être contrôlée. On peut donc utiliser une quantité élevée de lymphocytes T, par exemple plus de 10⁵ cellules CD3+ par kg (de patient greffé). Selon un mode de réalisation particulier, la composition à injecter pour le conditionnement du patient comprend des lymphocytes T modifiés obtenus par :
i. lymphaphérèse du donneur,
ii. transduction, de préférence le même jour, du gène codant une molécule permettant la destruction spécifique des lymphocytes T (par exemple un gène « suicide »),
iii. puis culture des lymphocytes T ainsi modifiés pendant dix à 21 jours, jusqu'à l'obtention d'environ 10⁸ cellules par kg de masse corporelle du patient.

Entre J-10 et J-3, J0 étant le jour de la greffe de cellules souches hématopoïétiques, la composition de lymphocytes T modifiés est administrée au patient par injection après un traitement lymphopéniant myéloablatif ou non myéloablatif.

Ce traitement peut se caractériser par une irradiation comprise entre 2 et 12 Gy. De plus cette irradiation peut être associée à l'administration de la cyclophosphamide (120mg/kg) combinée avec du busulfan (16mg/kg) ou l'irradiation peut être combinée avec du cyclophosphamide de la fludarabine et/ou de l'endoxan.

Miller et al, 2007, décrivent un traitement lymphopéniant non myéloablatif, qui peut être également utile. Ce traitement comprend une injection IV de cyclophosphamide 50mg/kg une fois à J-6 et J-5, et injection de fludarabine 25mg/m² de J-6 à J-2 (J étant le jour de la greffe des lymphocytes T). Powell et al , 2007; décrivent un autre protocole comprenant l'injection de fludarabine 25mg/m² pendant 5 jours et d'endoxan 60mg/kg/jours pendant 2 jours.

Puis, l'analogue de nucléoside est administré au patient de préférence par une ou plusieurs doses quotidiennes à partir de quelques heures jusqu'à 3 jours après l'injection de Lymphocytes T.

Les exemples et figures illustrent l'invention sans en limiter la portée.

### LEGENDE DES FIGURES

La Figure 1 est un diagramme montrant le pourcentage d'expression du marqueur H2Kb chez plusieurs groupes de souris ayant reçu des traitements de « conditionnement » différents à J+27 (J0 étant le jour de la greffe de cellules souches hématopoïétiques). Irr 7Gy signifie que les souris ont été soumises à une irradiation de 7Gy. GMO signifie greffe de moelle osseuse. GVC signifie Gancyclovir.
La Figure 2 est un diagramme montrant le pourcentage d'expression du marqueur H2Kb chez plusieurs groupes de souris ayant reçu des traitements de « conditionnement » différents à J+57. Irr 7Gy signifie que les souris ont été soumises à une irradiation de 7Gy. GMO signifie greffe de moelle osseuse. GVC signifie Gancyclovir.
La **Figure 3** décrit une étude du chimérisme en fonction de la dose d'irradiation à 1 mois.
Les **Figures 4** **et** **5** montrent l'impact des lymphocytes T exprimant le gène TK dans la prise de greffe.
La **Figure 6** illustre un protocole théraeutique avec conditionnement du receveur.

### EXEMPLES

Des études ont été réalisées sur des souris. Il a été utilisé comme souris receveuses des souris (B6xD2)F1 H2-^{bxd} et comme souris donneuses de cellules souches hématopoïétiques des souris C57BU6CD3e-/-. Dans cette expérience, les cellules souches hématopoïétiques proviennent de la moelle osseuse. Toutes les souris receveuses (B6xD2)F1 H2 ^{-bxd} ont été exposées à une irradiation de l'ordre de 7Gy. Il a été prélevé chez toutes les souris donneuses C57B1/6CD3e-/- une quantité de 5x10⁵ cellules souches hématopoïétiques issues de la moelle épinière. Un groupe de souris receveuse (B6xD2)F1 H2^{-bxd} a été soumis à une irradiation de 7Gy associée à l'injection d'une composition comprenant ±2x10⁶ lymphocytes T C57B1/6TK. Le deuxième groupe de souris receveuses (B6xD2)F1 H2^{-bxd} a subi une irradiation de 7Gy durant son « conditionnement » à J-3, sans injection de Lymphocytes T TK. L'ensemble des souris receveuses (B6xD2)F1 H^{2-bxd} ont été greffées avec une quantité de 5x10⁵ cellules souches hématopoïétiques issues de la moelle épinière des souris donneuses 72h après l'irradiation. Le groupe de souris (B6xD2)F1 H2^{-bxd} ayant reçu lors de son « conditionnement » la composition comprenant ±(2x10⁶) de lymphocytes T C57B1/6TK a été subdivisé en deux sous-groupes. L'un des sous-groupes a été exposé au Gancyclovir (analogue de nucléoside) à J-3 tandis que l'autre sous-groupe a reçu le Gancyclovir à J-1.

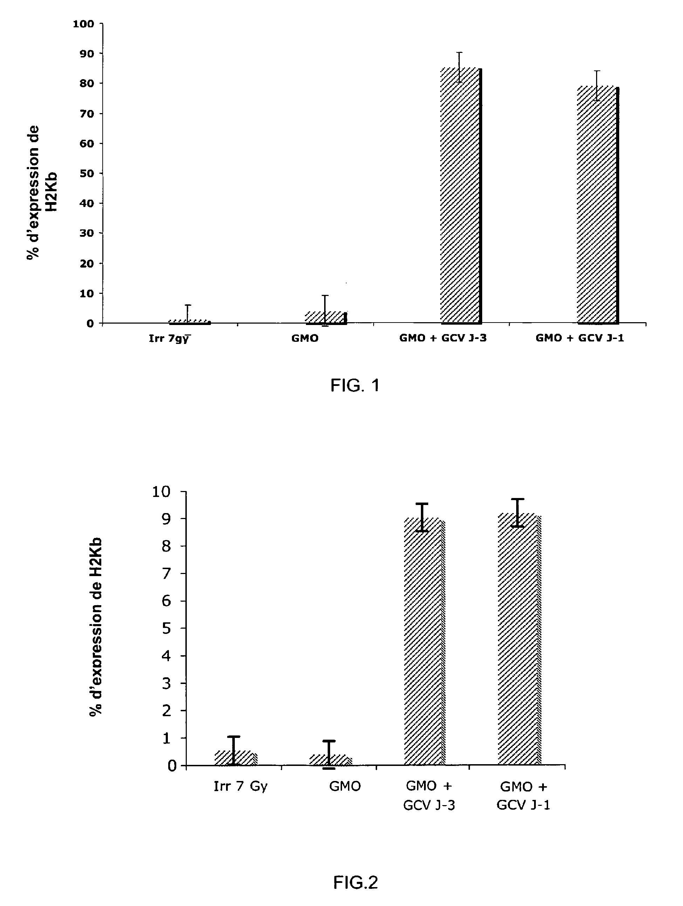

Pour étudier le chimérisme des souris receveuses à J+27 **(****Figure 1****)** et J+57 **(****Figure 2****),** J0 étant le jour de la greffe de cellules souches hématopïetiques, il a été mesuré le pourcentage d'expression de H2Kb (cellules d'origine du donneur) et H2Kd (cellules d'origine du receveur) Il existe alors une corrélation entre le pourcentage de H2kb et H2Kd exprimé et la prise de greffe. Plus le pourcentage d'expression de H2Kb est élevé, plus la prise de greffe est importante. Inversement, moins le pourcentage d'expression de H2Kd est élevé, moins la prise de greffe est importante.

Dans le cas des souris receveuses dont le conditionnement avant la greffe s'est limité à l'irradiation de 7Gy, il a été relevé un pourcentage d'expression du gène H2Kb de l'ordre de 0% à J+27 et de l'ordre de 5% à J+57 Ce pourcentage correspond à un rejet de la greffe.

Pour le groupe de souris receveuses (B6xD2)F1 H2^{-bxd} ayant reçu une injection de la composition de lymphocytes T C57B1/6TK et une injection de Gancyclovir à J-3, le pourcentage de H2Kb s'élevait à plus de 80% à J+27 et à quasi 100% à J+57.

Pour le groupe de souris receveuses (B6xD2)F1 H2^{-bxd} ayant reçu une injection de la composition de lymphocytes T C57B1/6TK et une injection de Gancyclovir à J-1, le pourcentage de H2Kb était de 80% à J+27 et et à quasi 100% à J+57à J+57. Ce pourcentage élevé d'expression de H2Kb correspond à une excellente prise de greffe. Aucun signe de GVH n'a été détecté.

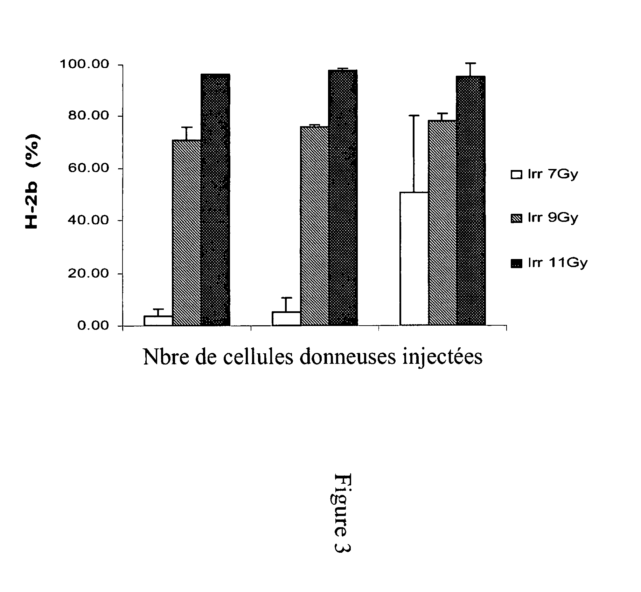

Dans une autre série d'expériences, les souris receveuses (B6xD2)F1 H2^{-bxd} ont été exposées à une irradiation variable allant de 7 à 11 Gy. Il a été prélevé chez toutes les souris donneuses C57B1/6CD3e-/- une quantité de cellules souches hématopoïétiques issues de la moelle épinière comprise entre 5x10⁵ et 2x10⁶ cellules. Les résultats sont présentés dans la Figure 3 et illustrent l'excellente prise de greffe chez les animaux traités. En particulier, on observe qu'à 9 et 11 Gy, il y a prise de greffe quasi complète quel que soit le nombre de cellules médullaires injectées.

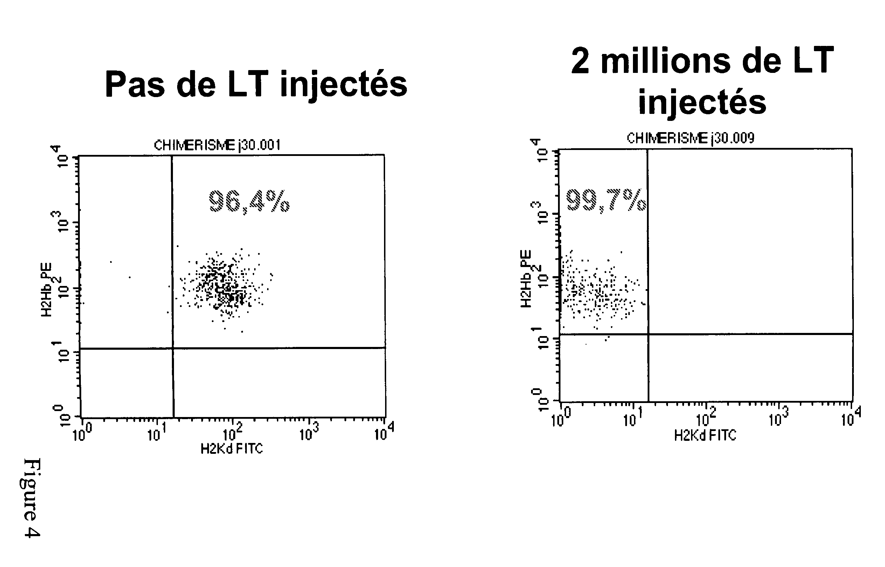

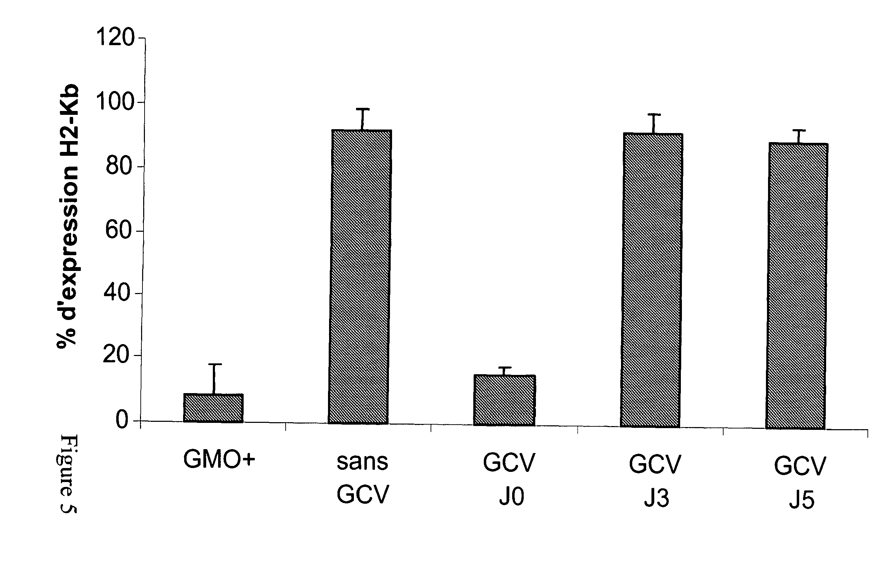

Dans une expérience supplémentaire, on a ajouté aux 2.10⁶ cellules de moelle osseuse injectées, 2.10⁶ cellules provenant des ganglions périphériques et contenant environ 80% de lymphocytes T. Comme le montre la figure 4, l'ajout des lymphocytes T allogéniques permet de passer à 99,7% de cellules d'origine du donneur, mais 100% des souris développent une GVH mortelle. En revanche, lorsque l'on ajoute des lymphocytes T provenant de souris transgéniques TK+ le jour de la greffe de moelle osseuse, et que l'on soumet les souris à un traitement par le ganciclovir (GCV) à J0, J3 ou J5 pendant 2 jours, il est possible d'améliorer considérablement la prise de greffe allogénique. Ainsi, comme le montre la **figure 5****,** sans GCV, la prise de greffe est quasi totale mais les souris meurent de GVH. La présence de GCV à JO permet un contrôle efficace de la GVH, et l'administration décalée de GCV (à J3 ou à J5) permet une prise de greffe quasi complète sans que les souris ne développent une GVH clinique.

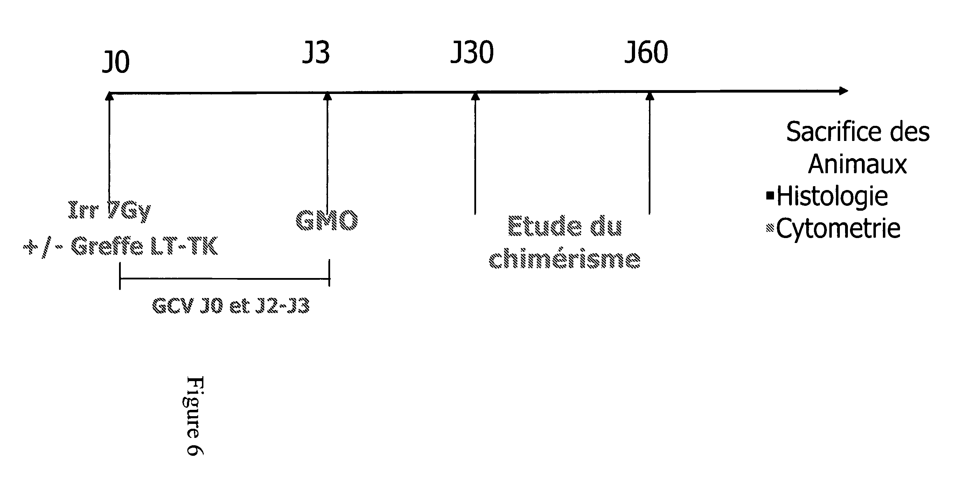

Un autre protocole de greffe est décrit dans la **Figure 6****.** Selon ce protocole, on décale le moment d'administration des cellules médullaires. Ainsi, le traitement débute par une étape de « conditionnement du receveur » par la présence de lymphocytes T alloréactifs pendant 3 jours. Puis, on élimine ces lymphocytes T par un traitement par le ganciclovir. On procède alors à la greffe de moelle osseuse.

Enfin, nous avons reproduit le même type d'expérience mais cette fois ci les cellules de la moelle, (BALB/c) les lymphocytes T TK+ (C57BL/6) et le receveur (C3H) proviennent de 3 fonds génétiques différents. Dans ces conditions, lorsque le GCV est administré à J2, nous avons pu obtenir une prise de greffe partielle (chimérisme mixte).

### Références bibliographiques

- Aversa et al, Treatment of High risk acute leukaemia with T-cell-depleted stem cells from related donors with one fully mismatched HLA haplotype. N Eng J Med, 1998; 339:1186-1193.
- Ciceri F, Bonini C, Marktel S, Zappone E, Servida P, Bemardi M, Pescarollo A, Bondanza A, Peccatori J, Rossini S, Magnani Z, Salomoni M, Benati C, Ponzoni M, Callegaro L, Corradini P, Bregni M, Traversari C, Bordignon C. 2007 Blood. 109(11):4698-707. Antitumor effects of HSV-TK-engineered donor lymphocytes after allogeneic stem-cell transplantation.
- Cohen et al, Suicide gene therapy of graft-versus-host disease: immune reconstitution with transplanted mature T cells. Blood, 2001; 98: 2071-2076.
- Danos, O. and Mulligan R. C. 1988. Safe and efficient generation of recombinant retroviruses with amphotropic and ecotropic host ranges. Proc. Natl. Acad. Sci. USA 85, 6460-6464.
- Kasid, A., Morecki, S., Aebersold, P., Cornetta, K., Culver, K., Freeman, S., Director, E. Lotze, M. T., Blaese, R. M. Anderson., W. F., and Rosenberg, S. A. 1990. Human gene transfer: characterization of human tumor-infiltrating lymphocytes as vehicles for retroviral-mediated gene transfer in man. Proc. Natl. Sci. USA 87, 473-477.
- Markowitz, D., Goff, S., and Bank, A. 1988. Construction and use of a safe and efficient amphotropic packaging cell line. Virology 167:400-406.
- Miller JS, Weisdorf DJ, Burns LJ, Slungaard A, Wagner JE, Vemeris MR, Cooley S, Wangen R, Fautsch SK, Nicklow R, Defor T, Blazar BR. 2007 Blood.110(7):2761-3, Lymphodepletion followed by donor lymphocyte infusion (DLI) causes significantly more acute graft-versus-host disease than DLI alone.
- Petersen et al, Alloreactivity of therapeutic principle in the treatment of hematologic malignancies. Danish Medical Bulletin, May 2007, 54: 112-39
- Ratanatharathorn V et al, Phase III study comparing methotrexate and tacrolimus (prograf, FK506) with methotrexate and cyclosporine for graft-versus-host disease prophylaxis after HLA-identical sibling bone marrow transplantation. Blood, 1998; 92: 2303-2314.
- Socie G et al, Acute graft-versus-host-disease. The Clinical Practice of stem-cell transplantation. Isis Medical Media: Oxford, 1998, pp595-618.
- Storb R et al, Methotrexate and cyclosporine versus cyclosporine alone for prophylaxis of graft-versus-host disease in patients given HLA-identical marrow for leukemia: long-term follow-up of a controlled trial. Blood, 1989; 73: 1729-1734.

## Revendications

1. Composition de lymphocytes T allogéniques, pour une utilisation dans le conditionnement d'un patient receveur d'une greffe de cellules souches hématopoïetiques, par injection de la composition audit patient receveur de 1 à 15 jours avant la greffe, lesdits lymphocytes T allogéniques exprimant une molécule permettant leur destruction spécifique.

2. Composition de lymphocytes T allogéniques, pour une utilisation dans le conditionnement d'un patient receveur d'une greffe de cellules souches hématopoïetiques selon la revendication 1, dans laquelle les lymphocytes T expriment un transgène permettant leur destruction spécifique.

3. Composition de lymphocytes T allogéniques, pour une utilisation dans le conditionnement d'un patient receveur d'une greffe de cellules souches hématopoïetiques selon la revendication 2, dans laquelle le transgène est un gène « suicide ».

4. Composition de lymphocytes T allogéniques, pour une utilisation dans le conditionnement d'un patient receveur d'une greffe de cellules souches hématopoïetiques selon la revendication 3, dans laquelle le gène « suicide » code pour une molécule capable de réagir avec un analogue de nucléoside pour conduire à la mort desdits lymphocytes T.

5. Composition de lymphocytes T allogéniques, pour une utilisation dans le conditionnement d'un patient receveur d'une greffe de cellules souches hématopoïetiques selon la revendication 4, dans laquelle ladite molécule codée par le gène « suicide » est une molécule apte à phosphoryler un analogue de nucléoside en une molécule monophosphatée, elle-même convertible par des enzymes cellulaires en nucléotide triphosphaté incorporable dans des acides nucléiques en cours d'élongation sous l'effet des polymérases avec pour effet l'interruption d'élongation des chaînes.

6. Composition de lymphocytes T allogéniques, pour une utilisation dans le conditionnement d'un patient receveur d'une greffe de cellules souches hématopoïetiques selon la revendication 5, dans laquelle ladite molécule codée par le gène « suicide » est la thymidine kinase du virus de l'herpès simplex de type 1.

7. Composition de lymphocytes T allogéniques, pour une utilisation dans le conditionnement d'un patient receveur d'une greffe de cellules souches hématopoïetiques selon l'une des revendications 1 à 6, dans laquelle lesdits lymphocytes T ne proviennent ni du donneur ni du receveur.

8. Composition de lymphocytes T allogéniques, pour une utilisation dans le conditionnement d'un patient receveur d'une greffe de cellules souches hématopoïetiques selon l'une des revendications 1 à 7, dans laquelle lesdites cellules souches hématopoïétiques sont des cellules souches hématopoïétiques issues de la moelle osseuse, du sang périphérique après mobilisation ou du sang de cordon ombilical.

9. Composition de lymphocytes T allogéniques, pour une utilisation dans le conditionnement d'un patient receveur d'une greffe de cellules souches hématopoïetiques selon l'une des revendications 1 à 8, dans laquelle l'injection des lymphocytes T est suivie par une destruction desdits lymphocytes T avant la greffe de cellules souches hématopoïetiques.

10. Composition de lymphocytes T allogéniques, pour une utilisation dans le conditionnement d'un patient receveur d'une greffe de cellules souches hématopoïetiques selon la revendication 9, dans laquelle les lymphocytes T expriment le gène de la thymidine kinase, et l'injection des lymphocytes T est suivie par une administration de gancyclovir ou acyclovir avant la greffe.

11. Composition de cellules souches hématopoïétiques d'un donneur, pour une utilisation dans le traitement d'un patient receveur nécessitant une telle greffe, ledit patient ayant été traité par injection de lymphocytes T allogéniques exprimant une molécule permettant leur destruction spécifique, de 1 à 15 jours avant la greffe.

## Claims

1. A composition of allogenic T lymphocytes for use in conditioning a recipient patient for a graft of haematopoietic stem cells, by injection of the composition to said recipient patient from 1 to 15 days before said graft, said allogenic T lymphocytes expressing a molecule allowing their specific destruction.

2. The composition of allogenic T lymphocytes for use in conditioning a recipient patient for a graft of haematopoietic stem cells, according to Claim 1, wherein the T lymphocytes express a transgene allowing their specific destruction.

3. The composition of allogenic T lymphocytes for use in conditioning a recipient patient for a graft of haematopoietic stem cells, according to Claim 2, wherein the transgene is a "suicide" gene.

4. The composition of allogenic T lymphocytes for use in conditioning a recipient patient for a graft of haematopoietic stem cells, according to Claim 3, wherein the "suicide" gene encodes a molecule capable of reacting with a nucleoside analogue in order to lead to the death of the said T lymphocytes.

5. The composition of allogenic T lymphocytes for use in conditioning a recipient patient for a graft of haematopoietic stem cells, according to Claim 4, wherein said molecule encoded by the "suicide" gene is a molecule capable of phosphorylating a nucleoside analogue to a monophosphate molecule, itself convertible by cellular enzymes to a triphosphate nucleotide that can be incorporated into nucleic acids during extension under the effect of polymerases, the effect being the interruption of chain extension.

6. The composition of allogenic T lymphocytes for use in conditioning a recipient patient for a graft of haematopoietic stem cells, according to Claim 5, wherein said molecule encoded by the "suicide" gene is thymidine kinase of the herpes simplex virus type 1.

7. The composition of allogenic T lymphocytes for use in conditioning a recipient patient for a graft of haematopoietic stem cells, according to one of Claims 1 to 6, wherein said T lymphocytes are obtained neither from the donor nor from the recipient.

8. The composition of allogenic T lymphocytes for use in conditioning a recipient patient for a graft of haematopoietic stem cells, according to one of Claims 1 to 7, wherein said haematopoietic stem cells are haematopoietic stem cells derived from the bone marrow, peripheral blood after mobilization or umbilical cord blood.

9. The composition of allogenic T lymphocytes for use in conditioning a recipient patient for a graft of haematopoietic stem cells, according to one of Claims 1 to 8, wherein the injection of the T lymphocytes is followed by destruction of said T lymphocytes before the graft of haematopoietic stem cells.

10. The composition of allogenic T lymphocytes for use in conditioning a recipient patient for a graft of haematopoietic stem cells, according to Claim 9, wherein the T lymphocytes express the thymidine kinase gene, and the injection of the T lymphocytes is followed by administration of gancyclovir or acyclovir before the graft.

11. A composition of haematopoietic stem cells of a donor, for use in treating a recipient patient requiring such grafting, wherein said patient has been treated by injection of allogenic T lymphocytes expressing a molecule allowing their specific destruction from 1 to 15 days before the grafting.

## Patentansprüche

1. Zusammensetzung allogener T-Lymphozyten zur Verwendung bei der Konditionierung eines Empfängers einer hämatopoetischen Stammzelltransplantation mittels Injektion der Zusammensetzung in besagten Empfänger 1 bis 15 Tage vor der Transplantation, wobei besagte allogene T-Lymphozyten ein Molekül exprimieren, das ihre spezifische Zerstörung erlaubt.

2. Zusammensetzung allogener T-Lymphozyten zur Verwendung bei der Konditionierung eines Empfängers einer hämatopoetischen Stammzelltransplantation gemäß Anspruch 1, wobei die T-Lymphozyten ein Transgen exprimieren, das ihre spezifische Zerstörung erlaubt.

3. Zusammensetzung allogener T-Lymphozyten zur Verwendung bei der Konditionierung eines Empfängers einer hämatopoetischen Stammzelltransplantation gemäß Anspruch 2, wobei das Transgen ein "Selbstmordgen" ist.

4. Zusammensetzung allogener T-Lymphozyten zur Verwendung bei der Konditionierung eines Empfängers einer hämatopoetischen Stammzelltransplantation gemäß Anspruch 3, wobei das "Selbstmordgen" für ein Molekül codiert, das in der Lage ist, mit einem Nukleosid-Analogon zu reagieren, um zum Tod besagter T-Lymphozyten zu führen.

5. Zusammensetzung allogener T-Lymphozyten zur Verwendung bei der Konditionierung eines Empfängers einer hämatopoetischen Stammzelltransplantation gemäß Anspruch 4, wobei besagtes, von dem "Selbstmordgen" codiertes Molekül ein Molekül ist, das in der Lage ist, ein Nukleosid-Analogon zu einem Monophosphat-Molekül zu phosphorylieren, das wiederum selbst von Zellenzymen in ein Nukleotid-Triphosphat umgewandelt werden kann, das in Nukleinsäuren im Verlauf der Elongation mithilfe der Polymerasen eingebaut werden kann, um den Abbruch der Kettenelongation zu bewirken.

6. Zusammensetzung allogener T-Lymphozyten zur Verwendung bei der Konditionierung eines Empfängers einer hämatopoetischen Stammzelltransplantation gemäß Anspruch 5, wobei besagtes, von dem "Selbstmordgen" codiertes Molekül die Thymidinkinase des Herpes-simplex-Virus Typ 1 ist.

7. Zusammensetzung allogener T-Lymphozyten zur Verwendung bei der Konditionierung eines Empfängers einer hämatopoetischen Stammzelltransplantation gemäß einem der Ansprüche 1 bis 6, wobei besagte T-Lymphozyten weder vom Spender noch vom Empfänger stammen.

8. Zusammensetzung allogener T-Lymphozyten zur Verwendung bei der Konditionierung eines Empfängers einer hämatopoetischen Stammzelltransplantation gemäß einem der Ansprüche 1 bis 7, wobei besagte hämatopoetische Stammzellen hämatopoetische Stammzellen sind, die vom Knochenmark, vom peripheren Blut nach Mobilisation oder vom Nabelschnurblut stammen.

9. Zusammensetzung allogener T-Lymphozyten zur Verwendung bei der Konditionierung eines Empfängers einer hämatopoetischen Stammzelltransplantation gemäß einem der Ansprüche 1 bis 8, wobei auf die Injektion der T-Lymphozyten eine Zerstörung besagter T-Lymphozyten vor der Transplantation der hämatopoetischen Stammzellen folgt.

10. Zusammensetzung allogener T-Lymphozyten zur Verwendung bei der Konditionierung eines Empfängers einer hämatopoetischen Stammzelltransplantation gemäß Anspruch 9, wobei die T-Lymphozyten das Thymidinkinase-Gen exprimieren und auf die Injektion der T-Lymphozyten eine Verabreichung von Gancyclovir oder Acyclovir vor der Transplantation folgt.

11. Zusammensetzung hämatopoetischer Stammzellen eines Spenders zur Verwendung bei der Behandlung eines Empfängers, der eine derartige Transplantation benötigt, wobei besagter Empfänger mittels Injektion allogener T-Lymphozyten, die ein Molekül exprimieren, das ihre spezifische Zerstörung erlaubt, 1 bis 15 Tage vor der Transplantation behandelt worden ist.
